(19) Europäisches Patentamt / European Patent Office / Office européen des brevets

(11) **EP 4 414 383 A1**

(12) **EUROPEAN PATENT APPLICATION**

(43) Date of publication:
**14.08.2024   Bulletin 2024/33**

(21) Application number: **23155907.1**

(22) Date of filing: **09.02.2023**

(51) International Patent Classification (IPC):
**C07K 14/72** (2006.01)     **C12N 15/63** (2006.01)
**C12Q 1/66** (2006.01)     **G01N 33/74** (2006.01)
**C12N 5/10** (2006.01)

(52) Cooperative Patent Classification (CPC):
**C07K 14/723; C12Q 1/66; G01N 33/74; G01N 33/76**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Ares Trading S.A.**
**1170 Aubonne (CH)**

(72) Inventors:
• **NEVELLI, Francesco**
**10010 Colleretto Giacosa (TO) (IT)**
• **DADONE, Aurora**
**10010 Colleretto Giacosa (IT)**
• **MARCONATO, Katia**
**10010 Colleretto Giacosa (IT)**

(74) Representative: **Merck Serono S.A.**
**Intellectual Property**
**Terre Bonne Business Park**
**Building Z0**
**Route de Crassier 1**
**1262 Eysins (CH)**

(54) **CELL LINES FOR EXPRESSION OF LHCG RECEPTOR**

(57)   The invention described herein relates to novel cell lines for the expression of a human LHCG receptor and for the expression of a human LHCG receptor together with a luciferase reporter gene. Also described are methods of preparing such cell lines. The cell line of the present invention expressing the LHCG receptor and the luciferase reporter gene is useable in a method for determining the presence and the biological activity of r-hLH and r-hCG samples.

EP 4 414 383 A1

**Description**

*Field of the Invention:*

Described herein are novel cell lines useful in the development of bioassays relevant in the manufacturing of recombinant human Luteinizing Hormone (r-hLH) and recombinant human Chorionic Gonadotropin (r-hCG). The first cell line expresses the human LHCG receptor (HEK 293T LHCGR) whereas the second cell line express both the human LHCG receptor and a luciferase reporter gene under the control of a cAMP response element (CRE) (HEK 293T LHCGR Luciferase).

*Background of the Invention:*

Bioassays are used to evaluate the drug potency before commercial products release on the market. To estimate drug potency, the assays should reflect or mimic the product's known mechanism of action (or part of it) that occurs in the human body, in order to reliably evaluate the product biological activity.

In vivo potency assays are methods in which animals are treated with Standard and Test materials and the relationship between product concentration and obtained response is evaluated to estimate the potency. Instead, cell-based (or in vitro) potency assays foresee the use of cell lines able to respond to specific ligands or infectious agents. The nature of cell lines can be different cells lines can be derived from tumors, immortalized as factor-dependent cell lines, or engineered cell lines transfected with appropriate target of interest. One of the main requirements is that these cell lines must be stable to guarantee assays reproducibility over time and reliable results delivery. Cellular responses to the protein of interest are related to the drug's mechanism of action and the duration of exposure. Some drug responses include cell proliferation, cell killing, cell differentiation, cytokine or other molecule secretion, intra-cellular response activation and enzyme activation.

Currently, the bioassays to determine presence and potency for recombinant human LH (r-hLH) and for recombinant human CG (r-hCG) involve in vivo assays including the use of animals. There is no alternative cell-based assay to be used in such test required for the manufacturing of these biological molecules. Therefore, there is a need for a cell line which can be used in such bioassays to determine the presence and potency of r-hLH or r-hCG in a sample.

*Summary of the Invention:*

The current invention is providing a solution to the lack of cell-based potency assays for determining the potency of r-hLH or r-hCG samples. The solution provides a new mammalian cell line that has been made to highly express the human LHCG receptor and which is couples to a reporter. As such, the binding of r-hLH or r-hCG to the human LHCG receptor on the new cell of the present invention allows the quantification of r-hLH or r-hCG biological activity.

In one embodiment there is provided a mammalian cell line for expressing a human LHCG receptor comprising: a.) a recombinant human LHCG receptor, comprising a nucleic acid molecule encoding the human LHCG receptor under the control of a promoter for protein expression, and b.) a reporter gene, comprising a nucleic acid molecule encoding for a luciferase reporter gene under the control of a cAMP Response Element (CRE).

In another embodiment there is provided a method of producing a human LHCG receptor in a mammalian cell comprising: a.) transfecting a mammalian cell with an isolated nucleic acid molecule encoding the LHCG receptor under the control of a promoter for protein expression, b.) adding a fist selectivity agent to the transfected cells of step a) to induce a stable expressing human LHCG receptor cell line (HEK 293-T LHCGR), c.) transfecting the mammalian cell of step b) with an isolated nucleic acid molecule encoding a luciferase reporter gene under the control of a cAMP response element (CRE), obtaining transfected cells, d.) adding a second selectivity agent to the stably expressing human LHCG receptor cells of step c) to induce cells stably expressing human LHCG receptor and the luciferase reporter, and e.) harvesting the cells stably expressing the human LHCG receptor and the luciferase reporter (HEK 293-T LHCGR Luciferase).

In yet another embodiment there is provided a method for biological activity quantification of r-hLH or r-hCG samples, comprising: a.) contacting the sample with cell culture of the mammalian cell expressing the human LHCG receptor and a luciferase reporter, b.) adding luminescence substrate for the luciferase enzyme to the cell culture, c.) detecting the luminescence of the cells, d.) quantifying the amount of luminescence, and e.) determining the potency of r-hLH or r-hCG samples.

*Drawings*

Figure 1: Evaluation of first transfection efficiency. A) HEK 293-T LHCGR vs HEK 293-T WT responses to stimulation with r-hLH. B) HEK 293-T LHCGR response to stimulation with r-hFSH.

Figure 2: LHR staining on HEK 293-T WT and HEK 293-T LHCGR luciferase. Data were acquired using BD FACS Aria Fusion and analyzed using Flowjo software.

Figure 3: Cellular behavior during a defined range of passages (p.17-p.41). A) Analysis of cellular viability, B) Analysis of cellular doubling. (A-B) Data are presented as mean $\pm$ SD of the three banks. Statistic was performed using linear regression analysis (A-B not significant). C) Real-time cell growth analyzed with Incucyte. Overlap between the growth curve (12500 cell/well) at p.17 and p.41. Data are presented as mean $\pm$ SD of the different replicates.

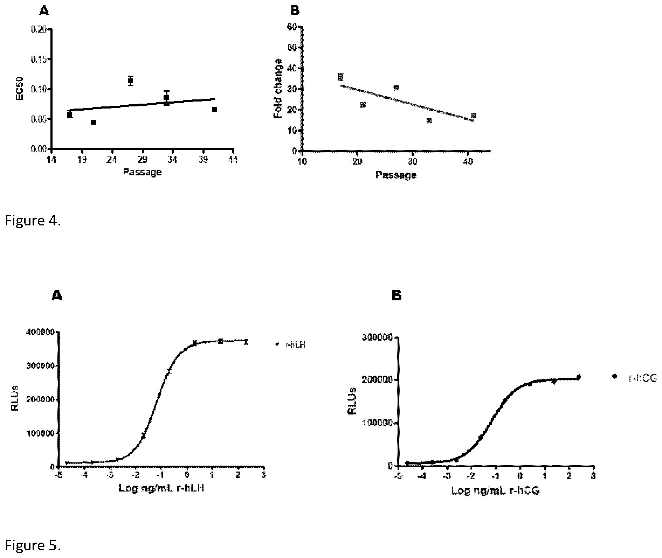

Figure 4: Monitoring of cell transfection stability during a defined range of passages (p.17-p.41). A) FRET analysis for LHCG receptor activity quantification. 4-parameter dose-response curves were generated and the EC50 from passage 17 to passage 41 was monitored. Data are presented as mean $\pm$ SD of the three banks. Statistic was performed using linear regression analysis (not significant). B) Luciferase reporter gene transcription activation with forskolin 4$\mu$M from passage 17 to passage 41. Data are presented as mean $\pm$ SD of the three banks. Statistic was performed using linear regression analysis (p=0.0013).

Figure 5: Representative RGA dose-response curves for potency quantification of r-hLH (panel A) and r-hCG (panel B) samples.

### *Detailed Description of the Invention:*

Currently, potency test for each new Lutropin Alpha and Choriogonadotropin alfa Drug Product batch release is performed with an *in vivo* method, using rats for quality control purpose. The *in vivo* method should be performed as required and described by the regulatory authorities and guidelines.

In view of the prevailing policies and ethical considerations the reliance on *in vivo* assays should be reduced where possible to be replaced with an alternative *in vitro* method. Such alternative method for testing r-hLH such as Lutropin Alpha Drug Product and r-hCG such as Choriogonadotropin alfa Drug Product can be an *in vitro* cell based method. A cell based analytical method for *in vitro* use for testing r-hLH and r-hCG requires an appropriate cell line expressing the LHCG receptor for binding of the r-hLH or r-hCG composition. Currently there is no cell line available on the market that can be suitable for the development of an in-vitro cell based analytical method, able to satisfy the quality control laboratory routine: the in-vitro method development would consider an easy procedure execution, the use of an easy and stable read-out, adopting a quantification reagent with available alternatives on the market.

The present invention provides such cell as well as a method of producing such cell line. Also provided is a method for *in vitro* testing r-hLH or r-hCG from a production batch using the new cell line.

The cell line of the present invention is based on a human embryonic kidney (HEK) cell line, more specifically a HEK 293-T.

The development of cell line and *in vitro* method were performed according to r-hLH and r-hCG mechanism of action in order to comply with guidelines and health authorities' requirements.

Cell line development started from HEK 293-T cells. The human origin cell line HEK 293-T was stably transfected with the human LHCG receptor. Afterward, a second transfection was performed on HEK 293-T LHCGR for the expression of the luciferase reporter gene.

The newly created cells will faithfully reproduce a part of the *in vivo* r-hLH and r-hCG mechanism of action: the first steps of the metabolic cascade produced by the receptor binding with the hormone.

The transfection processes were resulting in a new cell line of HEK 293 T cells expressing a human LHCG receptor and a luciferase reporter (HEK 293-T LHCGR Luciferase). The transfected cells were selected and expanded with consequent freezing of different cell banks. After cell banks creation, two different functional assays were developed with the aim to obtain a method able to preliminary assess the transfections efficiency under a functional point of view.

The first functional assay was developed for quantifying the cAMP, using FRET commercial kit, after cells stimulation, . In this way, the assay allowed the assessment of LHCG receptor activation in HEK 293-T LHCGR cells. Thanks to this assay it has been possible to appreciate the differences between transfected and non-transfected cells, confirming the expression of the LHCG receptor only in transfected cells.

The second functional assay was developed to assess the reporter gene transcription after intracellular cAMP accumulation, thanks to the inserted vector in HEK 293-T LHCGR cells during the second transfection process. This assay was carried out stimulating the cells with forskolin with consequent adenylate cyclase activation. Thanks to this stimulation, intracellular cAMP increased, and the reporter gene transcription should be evaluated in transfected cells, independently from LHCG receptor activation. Also in this case, it has been possible to observe the differences in luminescence production, after cells stimulation, between transfected and non-transfected cells. Thanks to this assay it has been possible to confirm the presence and the functionality of the luciferase reporter gene vector in HEK 293-T LHCGR Luciferase cell line.

A characterization study on HEK 293-T LHCGR Luciferase was performed during a defined range of passages, from passage 17 until passage 41. During this study, cells behavior was monitored assessing specific parameters like LHCG receptor expression in cells membrane, cell cycle, cells viability, cells proliferation (including cells doubling time and real-time cells proliferation monitoring). Also the previously developed functional assays were included in the characterization study, allowing the monitoring of first and second transfections stability over time.

The LHCG receptor expression in HEK 293-T LHCGR Luciferase membrane was assessed, in comparison with non-transfected cells. Thanks to these experiments it has been possible to confirm the presence of the LHCG receptor in transfected cells, obtaining a percentage of LHCGR positive cells around 70%.

The phenotypic and functional characterization of HEK 293-T LHCGR Luciferase cell line highlighted a comparable behavior among the three different cell banks created in the laboratory (MCB, WCB1 and WCB2). In fact, the results obtained for HEK 293-T LHCGR Luciferase demonstrated that cells presented stable viability and stable proliferation during the selected range of passages.

The receptor expression stability in cells membrane was indirectly tested during a defined range of passages by evaluating the cAMP pathway activated by LH interaction with its receptor, thanks to the developed functional assay. HEK 293T LHCGR Luciferase cells stimulated with scalar doses of r-hLH produced well-described curves. Moreover, the EC50 remained stable for all the cell banks for at least 24 passages, confirming the transfection stability and functionality over time.

For the second transfection stability assessment, HEK 293-T LHCGR Luciferase were stimulated with $4\mu$M of forskolin or left unstimulated. Moreover, during the analyzed 24 passages, it was noticed that the cell responses to the forskolin was maintained even if a progressive reduction in fold change was observed. The fold change is the ratio between the signal produced by forskolin stimulation and the unstimulated sample. Nevertheless, the lowest detected fold increase was higher than 10 that can be anyway considered more than acceptable for the assay purpose.

Beside all the activities related to the cell line development and characterization, the *in vitro* assay for r-hLH and r-hCG sample potency quantification was developed. The developed method procedure resulted very quick and easy; these characteristics ensure an optimal application of the assay in a quality control routine. The method demonstrated also to work properly as shown by the preliminary method linearity assessment. In fact, all the results were in accordance with the theoretical acceptance criteria.

Examples:

Culturing of HEK 293 T cells. The recommended cell culture medium is Dulbecco's Modified Eagle's Medium (DMEM) with the addition of 10% Fetal Bovine Serum (FBS). Cells doubling time is about 24-30 hours. Cells were cultured at $37\pm1$°C in a humidified atmosphere with $5\pm1$% CO2. All these cell characteristics are described in cells datasheet provided by the supplier, indeed the cell subculturing conditions have been identified in our laboratory.

Transfection of HEK 293T cells. The HEK 293T cell line was transfected with the LHCG receptor using an appropriate commercial vector (provided by Vector Builder) comprising a nucleic acid molecule encoding the human LHCG receptor. The vector, a mammalian gene expression vector, was 8097 bp. For a stable transfection, hygromycin B was used as a selection marker. Before transfection it was identified the minimum hygromycin B concentration able to interrupt HEK 293-T growth in 10 days performing a killing curve assay. The concentration of 200 $\mu$g/mL was identified as the best and used in all transfection experiments.

The vector encoding for LHCG receptor, was initially introduced into HEK 293-T cells using electroporation. The commercial reagents used for the transfection were included in the Amaxa 4D with the commercial kit (Lonza, V4XC-2024).

The medium used during all the transfections was the OptiMEM (Life Technologies, 31985). Different experimental conditions of transfection with electroporation were tested, using different amounts of vector DNA. The transfection process, as well as the different experimental conditions, were performed according to the protocol suggested by the supplier. 24 hours after transfection, the antibiotic selection was added to the cells with the aim to select only the population able to resist to the antibiotic concentration, identified during the kill curve experiment. Resistant cells were expanded and different cell banks belonging to each transfection condition were frozen. The usual approach for the creation of a cell bank foresees the freezing of a Master Cell Bank (MCB). After the expansion of the MCB, a Working Cell Bank (WCB) is created. Banks creation has the aim to ensure the availability of a sufficient number of cells during the development and the execution of an assay. The bank generation depends on cells growth characteristics, while the size of a bank depends on the number of cells required for each assay and how often the assay will be performed.

First transfection efficiency evaluation. In order to evaluate transfection efficiency, transfected cells were stimulated with r-hLH and cAMP production was quantified using a commercial kit based on Fluorescence Resonance Energy Transfer (FRET). The FRET commercial kit used to quantify cAMP was provided by Cisbio. The kit includes a cAMP coupled with d2 (acceptor) and an antibody anti-cAMP conjugated with a cryptate (donor, Europium). The FRET is detected by a plate reader equipped with the Homogeneous Time Resolve Fluorescence (HTRF) detector technology. All the experiments were performed according to the datasheet provided by the kit supplier.

For data interpretation, the ratio between acceptor and donor emission signals must be calculated for each individual well using the following formula:

$$\text{Ratio} = (\text{Signal 665 nm})/(\text{Signal 620 nm}) * 10000.$$

All the raw data (Ratio) produced with the FRET kit, were analyzed and graphed with the statistical software GraphPad Prism. The statistical analysis performed was the Ratio Log transformation and creation of the 4PL curve.

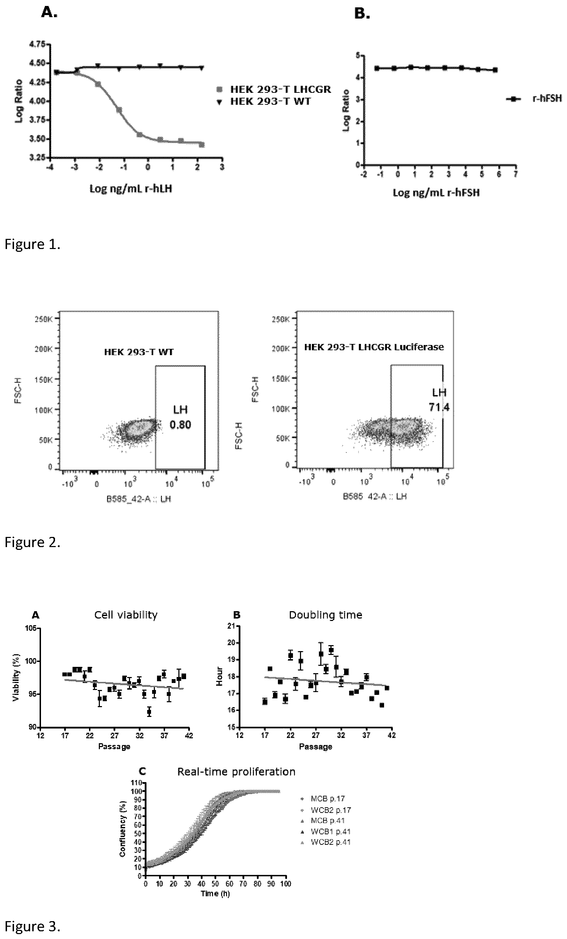

Cells were plated in a 96 well plate at the same concentration and stimulated with different amount of r-hLH, creating a dose-response curve. As negative control, HEK 293-T without transfection were also stimulated. After 30 minutes of incubation, FRET reagents were added in each well of the plate. The plate was again incubated under shaking conditions for 60 minutes and finally the results were acquired at the plate reader. In Figure 1 panel A it was possible to observe how the responses produced by the two cell lines were different. Transfected cells respond to r-hLH stimulation producing a well described dose-response curve whereas non-transfected HEK 293-T were not able to produce a dose-related response after stimulation, confirming the absence of the receptor in cells membrane.

Moreover, HEK 293-T LHCGR response specificity was also verified stimulating the cells with an analogue hormone of r-hLH: r-hFSH. In Figure 1 panel B is shown that HEK 293-T LHCGR cell response to r-hFSH stimulation was absent, confirming the inability of HEK 293-T LHCGR cells to increase the intracellular cAMP accumulation after stimulation with r-hFSH.

Transfection of HEK 293T LHCGR cells. A second transfection was performed on HEK 293-T LHCGR previously transfected with LHCG receptor vector to generate a reporter gene cell line. The selected commercial vector (Luc2P/CRE CS188015, Promega) contained a cAMP response element (CRE) that drives the transcription of the luciferase reporter gene luc2P (Photinus pyralis). The vector backbone contains a mammalian selectable marker for puromycin resistance. Before transfection it was identified the minimum puromycin concentration able to interrupt HEK 293-T LHCGR growth in 10 days performing a killing curve assay. The concentration of 0.5 $\mu$g/mL was identified as the best and used in all transfection experiments.

The vector encoding for luciferase reporter gene was introduced into HEK 293-T LHCGR cells using FuGENE HD Transfection Reagent (Promega, E2311). The medium used during the transfection was the OptiMEM (Life Technologies, 31985). Different experimental conditions of transfection were tested, using different amounts of vector DNA and different ratio of reagent and vector DNA.

The transfection process, as well as the different experimental conditions, were performed according to the protocol suggested by the supplier. 24 hours after transfection, the antibiotic selection was added to the cells with the aim to only select the population able to resist to the antibiotic concentration identified during kill curve experiment. Resistant cells were expanded and different cell banks, belonging to each transfection technique, were frozen.

Second transfection efficiency evaluation. In order to assess the second transfection efficiency, a Reporter Gene Assay (RGA) was performed. This assay was suggested in the Promega vector datasheet. HEK 293-T LHCGR Luciferase cells were stimulated with forskolin. As described in literature, forskolin is a diterpene able to activate the adenylate cyclase (AC) allowing the increasing of intracellular cAMP in a wide variety of cell types. Exploiting forskolin ability, the RGA assay enables the evaluation of Reporter Gene Transcription in response to cAMP accumulation without involving the LHCG receptor. The luminescence emitted after Reporter Gene Transcription, was quantified using reagents available on the market.

HEK 293-T LHCGR Luciferase and HEK 293-T without transfection were seeded in a 96 well plate and stimulated with different concentration of forskolin. After incubation, the ONE-Glo EX Luciferase assay system (Promega, E8120) was added in each well and, after 10 minutes shaking, results were acquired using the plate reader.

In Table 1 are shown the luminescence values (expressed as Relative Lights Units, RLUs) obtained for each well after plate reading. As expected, HEK 293-T without transfection (WT) didn't produce a luminescence response to forskolin stimulation due to the absence of the receptor pathway. HEK 293-T LHCGR Luciferase responses demonstrated to be dose-related. The highest RLUs values were obtained in correspondence of 40 $\mu$M of forskolin. Also at 4 $\mu$M the luminescence values can be considered suitable for the assay purpose. At 400 $\mu$M the obtained response was lower than at 40 $\mu$M because the cells reached the plateau of their maximal response. These data confirmed the success of the second transfection.

| [Forskolin] | HEK 293-T WT | | | HEK 293-T LHCGR Luciferase | | |
|---|---|---|---|---|---|---|
| 400 µM | 227 | 130 | 124 | 205580 | 197090 | 195210 |
| 40 µM | 256 | 133 | 104 | 374090 | 378740 | 387970 |
| 4 µM | 285 | 133 | 73 | 310180 | 299010 | 303780 |
| 0,4 µM | 177 | 101 | 86 | 65001 | 62733 | 72165 |
| 0,04 µM | 124 | 88 | 68 | 7547 | 6804 | 7636 |
| 0,004 µM | 92 | 61 | 62 | 4221 | 4333 | 3899 |

Table 1. Luminescence values expressed as RLUs obtained for each well after plate reading.

HEK 293-T LHCGR Luciferase: cell line characterization. Cell line characterization included all the activities related to the evaluation of determined cells characteristics. The aim of the study was to monitor and evaluate different parameters, in order to investigate cell line behavior over time. Cell line behavior evaluation is important to monitor cells viability, proliferation rate, target receptor expression and functional performances for a determine number of cell passages. The monitored parameters for HEK 293-T LH Luciferase cells were:

Presence of LHCG receptor expression in cells membrane,

Cells viability,

Cells proliferation (doubling time and real-time cell growth verification),

First transfection stability,

Second transfection stability.

To give consistency to the analysis and to generate results representative of the cell line and not related to a specific cell bank, three vials belonging to three different cell banks were tested: MCB, WCB1 and WCB2. Cells were monitored for a long-time starting from passage 17 until passage 41.

Each parameter was assessed during the defined range of passages. The variability among the three vials was calculated using coefficient of variation (CV%). The three vials were analyzed together to calculate mean and standard deviation (SD). Data were analyzed using linear regression analysis at the 95% of regression significance (p-value <0.05). All statistical analyses were performed using Graph Pad Prism.

LHCG receptor expression assessment in cells membrane. The percentage of transfected cells expressing the human LHCG receptor in their membrane was assessed. For this scope, cells were stained and analyzed by FACS Aria Fusion instrument (BD Biosciences). Data analysis for staining experiments was performed using FlowJo software. The cell lines used for the staining were HEK 293-T LHCGR Luciferase MCB and the HEK-293-T WT without transfection. A primary LHR conjugated antibody (LHCGR/1417 [PE], Novusbio) was used for the staining. The staining protocol was developed taking advantage of cells fixation and permeabilization before the antibody addition.

Fixation buffer (FB) was prepared diluting methanol 1:1 in PBS 1X and stored at room temperature. Two different wash buffers (WB1 and WB2) were used composed by PBS 1X and 2% fetal bovine serum. WB2 included also a 0.1% Tween20 reagent. $1*10^6$ cells were resuspended in WB1, transferred in tubes and centrifuged at 300 x g for 6 minutes. After supernatant removal, 1 mL of FB was added in each tube and incubated for 10 minutes at -20°C. After incubation, cells were washed with 2mL of WB2. The staining was performed in two different steps, using the primary LHR conjugated antibody. The first step foreseen the addition in each tube of the FcR blocking reagent (Miltenyi biotec, 130-059-901) diluted in WB2 and incubated for 5 minutes at room temperature, before the addition of the antibody. The antibody was diluted in WB2 and incubated for 30 minutes at room temperature. After incubation, cells were washed with WB2 and resuspended in WB2. Results were acquired at FACS instrument.

As shown in the figure 2, some differences between non-transfected cells (HEK 293-T WT) and transfected cells (HEK 293-T LHCGR Luciferase) were visible. In fact, HEK 293-T LHCGR Luciferase presented a 71% of cells positive for the LHCG receptor versus the 0.89% positive stained cells in HEK 293-T WT sample. This staining experiment confirmed the success of the first transfection.

HEK 293-T LHCGR Luciferase viability. Cell line viability measurement was performed using Nucleocounter instrument (Chemometec). Results highlighted a viability >90% after thawing that remained stable during the range of passages analyzed (Figure 3A). The mean and the SD of the 3 banks were calculated and reported in Figure 3A. The average viability was estimated at around 96.55%±1.61% (mean ± SD) with a slight but not significant downward trend during the range of passages.

HEK 293-T LHCGR Luciferase proliferation evaluation. Cell proliferation evaluation allowed to set-up the best conditions for cells subculturing in terms of quantity of seeded cells, thanks to the cells doubling time calculation. At all the cell passages, the number of total cells was assessed using NucleoCounter (Chemometec) and the doubling time was calculated using the following equation:

$$Doubling\ time = \frac{duration\ (hour) * \log(2)}{\log(Final\ concentration) - \log(Initial\ concentration)}$$

The mean and the SD of the 3 banks were calculated and reported in Figure 3B. The doubling time was estimated at around 17.71 ± 1.01 hours (mean ± SD) with a slight but not significant downward trend during the range of passages.

For real-time proliferation evaluation, a determined number of cells was seeded in a 96 well plate (100.000 cell/well, 50.000 cell/well, 25.000 cell/well, 12.500 cell/well, 6.250 cell/well e 3.125 cell/well). Cells were incubated for 4 days into the Incucyte instrument (Sartorius). Pictures from each well were acquired every hour. A confluence mask was created on Incucyte analysis tool to calculate the occupied area (% confluence) of cell images over time and generate cell growth curves. The overlap between the growth curves (12500 cell/well) at passage 17 and passage 41 confirmed that no alteration in cell proliferation occurs during the range of passages (Figure 3C).

First transfection stability. The LHCG receptor transfection stability was indirectly evaluated through the quantification of the cAMP produced after r-hLH interaction with its receptor. HEK 293T LHCGR Luciferase, were stimulated with scalar doses of r-hLH in a 96-well plate and the quantity of cAMP present in each well was quantified using the FRET commercial kit. A 4-parameter dose-response curve was generated for each test using GraphPad Prism (v.8) software. The EC50 was chosen as the parameter to be monitored over time. The mean and CV% among the three banks were calculated and reported in figure 4A. As shown in the figure 4A the EC50 remained stable during the analyzed range of passages.

Second transfection stability. HEK 293-T LHCGR Luciferase were stimulated with forskolin 4μM or left unstimulated. Results were reported as a fold change of treated cells over unstimulated control. All %CVs were calculated among fold changes obtained for the three banks and resulted below 25%. The mean and the SD of the 3 banks were calculated and used to generate the graphs in Figure 4B. With the passage increase it was noticed a progressive reduction in luminescence values producing a significant fold change decrement (Figure 4B).

Reporter Gene Assay: method development. A Reporter Gene Assay was set-up for r-hLH and r-hCG potency assessment using HEK 293-T LHCGR Luciferase cell line. The developed method was able to reproduce a portion of the r-hLH and r-hCG mechanism of action: the first steps of the metabolic cascade produced by the receptor binding with the hormone. The assay principle foreseen the cells plating at the same concentration (40000 cell/well) in each well of a 96 well plate and their stimulation with a starting dose of 200ng/mL of r-hLH or 250ng/mL of r-hCG, applying a 1:10 dilution step. After 4-hour incubation, the commercial luminescence substrate was added into each well and, after shaking, results were acquired using a plate reader. All the produced data were analyzed using GraphPad Prism software, interpolating the values of RLUs obtained in each well with the log-transformed nanograms/mL of hormone used at each dose.

In Figure 5 are reported the obtained dose-response curves for r-hLH (panel A) and r-hCG (panel B). Transfected cells response to both hormones' stimulation producing well described dose-response curves.

Two preliminary method linearity were carried out to assess methods performance using r-hLH and r-hCG samples. the feasibility of method linearity was evaluated testing in triplicate different theoretical relative potency levels: 60%, 77%, 100%, 120%, 144%. These different potency levels were calculated starting from the concentration of 200 ng/mL of r-hLH and 250ng/mL of r-hCG. The starting concentration for both samples was considered as standard. For each sample it was calculated the relative potency (RP%) result.

Relative Potency (RP%)

$$RP\% = (EC50\ Standard)/(EC50\ Sample)*100$$

Linearity results were statistically analyzed using JMP software. JMP analysis included the generation of a graph reporting on the x axes the log Theoretical Relative Potency of tested samples (60% - 77% - 100% - 120% - 144%), and on the y axes the log Measured Relative Potency of the tested samples. A common approach to evaluate the goodness of fit of the obtained regression line is to evaluate different parameters as R2, the intercept confidence interval and the slope confidence interval. Thanks to the gained experience following multiple method validations in the laboratory, it was possible to define approximated acceptance criteria for all the parameters (Table 2).

Table2: Summary of linearity statistical analysis using both r-hLH and r-hCG.

| Parameter | Acceptance criteria | Results r-hLH | Results r-hCG |
|---|---|---|---|
| $R^2$ | ≥0.95 | 0.97 | 0.96 |
| Slope confidence interval | Encompass 1 | 0.93 - 1.16 | 0.97-1.19 |
| Intercept confidence interval | Encompass 0 | -0.74 - 0.32 | -0.90-0.13 |

**Claims**

1. A mammalian cell for expressing a human LHCG receptor and a luciferase reporter gene comprising:

   a. a human LHCG receptor, comprising a nucleic acid molecule encoding the human LHCG receptor under the control of a promoter for protein expression, and
   b. a reporter gene, comprising a nucleic acid molecule encoding for a luciferase reporter gene under the control of a cAMP Response Element (CRE).

2. The mammalian cell of claim 1, wherein the mammalian cell is a mammalian cell culture.

3. The mammalian cell of claim 2, wherein the mammalian cell culture is an adherent cell culture.

4. The mammalian cell of any one of claims 1-3, wherein the promotor for protein expression is a strong promoter for high level protein expression, such as CAG.

5. The mammalian cell of any one of claims 1-4, wherein:

   a. the nucleic acid molecule encoding the human LHCG receptor is a vector comprising the LHCG receptor gene and the gene for hygromycin resistance,
   b. the nucleic acid molecule encoding for the luciferase reporter gene is a vector comprising the luciferase reporter gene under the control of the cAMP Response Element (CRE) and a gene for puromycin resistance.

6. The mammalian cell of any one of claims 1-5, wherein the mammalian cell is a human embryonic kidney (HEK) 293 cell.

7. The mammalian cell line of claim 6, wherein the HEK 293 cell is a HEK 293 T cell.

8. A method of producing a human LHCG receptor and a luciferase reporter gene in a mammalian cell comprising:

   a. transfecting a mammalian cell with an isolated nucleic acid molecule encoding the LHCG receptor under the control of a promoter for protein expression,
   b. adding a fist selectivity agent to the transfected cells of step a) to induce a stable expressing human LHCG receptor cell line,
   c. transfecting the mammalian cell of step b) with an isolated nucleic acid molecule encoding a luciferase reporter gene under the control of a cAMP response element (CRE), obtaining transfected cells,
   d. adding a second selectivity agent to the stably expressing human LHCG receptor cells of step c) to induce cells stably expressing human LHCG receptor and the luciferase reporter, and
   e. harvesting the cells stably expressing the human LHCG receptor and the luciferase reporter.

9. The method of claim 8, wherein the first selectivity agent is hygromycin and the second selectivity agent is puromycin.

10. The method of claim 8 or 9, wherein the promoter for expression of protein is a strong promoter for high level protein expression, such as CAG.

11. A method of determining the biological activity of r-hLH or r-hCG samples, comprising

a. contacting the sample with cell culture of the mammalian cell of claims 1-7,
b. adding luminescence substrate for the luciferase enzyme to the cell culture,
c. detecting the luminescence of the cells,
d. quantifying the amount of luminescence, and
e. determining the biological activity of r-hLH or r-hCG samples.

A.

B.

Figure 1.

Figure 2.

Figure 3.

Figure 4.

Figure 5.

Europäisches
Patentamt

European
Patent Office

Office européen
des brevets

# EUROPEAN SEARCH REPORT

Application Number

EP 23 15 5907

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Jia Xiao-Chi: "Luminescence Luteinizing Hormone/Choriogonadotropin (LH/CG) Bioassay: Measurement of Serum Bioactive LH/CG during Early Pregnancy in Human and Macaque'", ,<br>1 December 1993 (1993-12-01), pages 1310-1316, XP093061920,<br>Retrieved from the Internet:<br>URL:https://academic.oup.com/biolreprod/article-pdf/49/6/1310/10538143/biolreprod1310.pdf<br>[retrieved on 2023-07-07]<br>* abstract * | 1-11 | INV.<br>C07K14/72<br>C12N15/63<br>C12Q1/66<br>G01N33/74<br>C12N5/10 |
| X | MINEGISHI Y ET AL: "Quantitative bioassays for measuring biologically functional gonadotropins based on eel gonadotropic receptors",<br>GENERAL AND COMPARATIVE ENDOCRINOLOGY, ACADEMIC PRESS, US,<br>vol. 178, no. 1,<br>27 April 2012 (2012-04-27), pages 145-152, XP028500646,<br>ISSN: 0016-6480, DOI:<br>10.1016/J.YGCEN.2012.04.030<br>[retrieved on 2012-05-09]<br>* abstract * | 1-11 | |
| X | US 2009/317858 A1 (HANSON BONNIE JEAN [US]) 24 December 2009 (2009-12-24)<br>* claims 1,9,10,12 *<br>-/-- | 1-11 | TECHNICAL FIELDS SEARCHED (IPC)<br><br>C07K<br>C40B<br>G01N<br>C12N<br>C12Q |

The present search report has been drawn up for all claims

| Place of search | Date of completion of the search | Examiner |
|---|---|---|
| Munich | 7 July 2023 | Griesinger, Irina |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
...............................................................
& : member of the same patent family, corresponding
document

EPO FORM 1503 03.82 (P04C01)

Europäisches
Patentamt

European
Patent Office

Office européen
des brevets

# EUROPEAN SEARCH REPORT

Application Number

EP 23 15 5907

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LAURA H HEITMAN ET AL: "Determination of different putative allosteric binding pockets at the lutropin receptor by using diverse drug-like low molecular weight ligands", MOLECULAR AND CELLULAR ENDOCRINOLOGY, ELSEVIER IRELAND LTD, IE, vol. 351, no. 2, 9 January 2012 (2012-01-09), pages 326-336, XP028459876, ISSN: 0303-7207, DOI: 10.1016/J.MCE.2012.01.010 [retrieved on 2012-01-18] * abstract * | 1-11 | |
| X | AIZEN JOSEPH ET AL: "Experimental and computational study of inter- and intra-species specificity of gonadotropins for various gonadotropin receptors", MOLECULAR AND CELLULAR ENDOCRINOLOGY, vol. 364, no. 1, 30 August 2012 (2012-08-30), pages 89-100, XP028945880, ISSN: 0303-7207, DOI: 10.1016/J.MCE.2012.08.013 * abstract * * item 2.3 * | 1-11 | |

TECHNICAL FIELDS
SEARCHED      (IPC)

The present search report has been drawn up for all claims

| Place of search | Date of completion of the search | Examiner |
|---|---|---|
| Munich | 7 July 2023 | Griesinger, Irina |

EPO FORM 1503 03.82 (P04C01)

## ANNEX TO THE EUROPEAN SEARCH REPORT
## ON EUROPEAN PATENT APPLICATION NO.

EP 23 15 5907

This annex lists the patent family members relating to the patent documents cited in the above-mentioned European search report.
The members are as contained in the European Patent Office EDP file on
The European Patent Office is in no way liable for these particulars which are merely given for the purpose of information.

07-07-2023

| Patent document cited in search report | Publication date | Patent family member(s) | Publication date |
|---|---|---|---|
| US 2009317858 A1 | 24-12-2009 | US 2007218456 A1 | 20-09-2007 |
| | | US 2009317858 A1 | 24-12-2009 |
| | | WO 2007092938 A2 | 16-08-2007 |

EPO FORM P0459

For more details about this annex : see Official Journal of the European Patent Office, No. 12/82